# EUROPEAN PATENT APPLICATION

(11) **EP 2 196 175 A1**
(43) Date of publication of application: **16.06.2010**
(21) Application number: 08021664.1
(22) Date of filing: 12.12.2008
(51) Int. Cl.: A61F 2/90, A61F 2/86

(54) **Covered toroid stent and methods of manufacture**

(71) Applicant: Abbott Laboratories Vascular Enterprises Limited, Terrace, Dublin 2 (IE)
(72) Inventor: Grandt, Axel, 72479 Strassberg (DE)
(74) Representative: Peters, Hajo

(57) **Abstract**

An expandable stent (1) for implantation in a body lumen, such as coronary artery, and methods for manufacturing such a stent are provided, whereby the stent comprises at least one radially expandable cylindrical crown (3) covered with a polymeric tube (12). The at least one crown is generally aligned on a longitudinal axis of the stent and connected to at least one further stent segment with highly flexible connectors (4) formed from the same or different polymeric material as the crown covering material. In a preferred embodiment the cylindrical crown is loaded or coated with a therapeutic active agent.

## Description

### BACKGROUND OF THE INVENTION

This invention relates to expandable endoprosthesic devices, generally called stents, which are adapted to be implanted into a patient's body lumen, such as blood vessel, to maintain the patency thereof. These devices are useful in the treatment of artherosclerotic stenosis in blood vessels.

Stents are generally tubular-shaped devices which function to hold open a segment of a blood vessel, coronary artery, or other anatomical lumen. They are particularly suitable for use to support and hold back a dissected arterial lining which can occlude the fluid passageway therethrough.

Numerous stent designs are known in the art, of which self-expanding and balloon-expandable stents are the predominant commercially available types. Self-expanding stents, such as described in U.S. Pat. No. 4,580,568 to Gianturco generally provide good crush resistance and a certain axially flexibility, thus permitting delivery through tortuous anatomy, but provide lower radial strength once deployed. Balloon-expandable stents, such as typified by Palmaz in U.S. Pat. No. 4,739,762 provide high radial strength, but tend to have increased axial rigidity that affects deliverability through tortuous vessels. Also combinations of stents having self-expanding portions as well as balloon-expandable portions have been described to combine the advantages of both stent types for example in bifurcations.

Generally speaking, most prior art intravascular stents are formed from a metal such as stainless steel, which is balloon expandable and plastically deforms upon expansion to hold open a vessel or of a shape-memory metal like Nitinol that shows superelastic characteristics and self-expands upon delivery into the bodily lumen. A common drawback of metal stents is their electrically active behaviour and the exposure of metal to the blood resulting in reduced biocompatibility. Other types of prior art stents may be formed from a polymer or known stents are formed from a metal coated with a polymer by spraying or dipping. These polymeric coatings may also contain pharmaceutically active agents as e.g. the coating of the drug-eluting stent described in EP Pat. No. 0970711. Though polymeric coatings applied by spraying or dipping provide good biocompatibility, there is always the risk of rupture or loss of the coating once the stent is expanded and thus leading to loss of particles in the vessel.

It therefore continues to be a need for highly flexible stents with a high radial force and excellent biocompatibility and the capability for loading a pharmaceutically active substance. The present invention satisfies these needs.

### SUMMARY OF THE INVENTION

The present invention is directed to an expandable stent for implantation in a body lumen, such as a coronary artery. The stent consists of at least two radially expandable cylindrical tubular stent elements generally aligned to each other on a longitudinal axis of the stent. At least one stent element is a cylindrical crown comprising a metallic core or torus and a polymeric cover over the core, the cylindrical crown being attached to the at least one second stent element by at least one connector which is also formed from a polymeric material.

In a preferred embodiment of the present invention the stent comprises a plurality of cylindrical crowns which are aligned along a longitudinal axis of the stent and attached to each other by at least one polymeric connector. Each crown comprises a metallic core and a polymeric cover over the core.

The polymeric connectors provide longitudinal and flexural flexibility of the stent to facilitate delivery through tortuous body lumens and the crowns maintain sufficient radial strength to maintain the patency of a vessel and to resist collapse, whereas the polymeric cover of the crowns provides improved biocompatibility as well as vast capabilities for both loading and eluting of pharmaceutically active substances.

The cylindrical crowns can be formed with undulations having peaks and valleys generally formed as U, V or W members. The peaks of each cylindrical crown can be axially aligned with the peaks and valleys of each adjacent cylindrical crown to provide the desired flexibility. In another embodiment the peaks and valleys of adjacent cylindrical crowns can be positioned laterally offset to each other to allow excellent bending of the stent.

Each cylindrical crown comprises a metallic core like a wire, band or tube which determines the shape of the crown as well as provides the radial strength of the stent. The metallic core is covered with a polymeric cover enclosing the metal core completely. In a preferred embodiment the polymeric cover is made from a polymer tube which is imposed on the metallic core in a loose-fitted way. In another embodiment the polymeric cover may also be sintered onto the metallic core to result in a tight fit of polymeric cover and metallic core.

The polymeric cover can be made from various polymers including but not limited to polyurethanes, polyesters, polyamides, copolymers, block copolymers and blends or multi-layers thereof. Further, the cover may be liquid tight and uniform or can be micro-porous, wherein the pores can be material inherent like, e.g. in ePTFE membranes or spun, knitted or weaved membranes or the pores can be created by punching holes into the membranes by suitable means, like e.g. laser.

In a preferred embodiment the polymeric cover is loaded with a pharmaceutically active substance that may be eluted into the vessel subsequent to deployment of the stent in a controlled manner.

Further, the present invention is directed to a method of forming a stent. A metallic core like a wire, a tube, or a band as well as a polymeric sheath, preferably a tube, are provided. In the first step the polymeric sheath or tube is imposed on the metallic core to give a polymeric cover. A circular crown is formed of the covered metallic core by first connecting the free ends of the metallic core to form a ring-shape; and secondly by connecting the free ends of the polymeric cover to form a ring-closed structure around the core. Once the cylindrical crown is manufactured the crown is aligned to at least one other stent element along a longitudinal axis of the stent, and the crown is attached to the at least one other stent element by at least one polymeric connector.

The stent of the present invention provides a highly flexible but radially strong, extremely biocompatible and electrically neutral structure which can be easily loaded with a therapeutic agent prior to implantation but which also can also be reloaded with a pharmaceutically active agent post implantation.

It is to be recognized that the stent elements or crowns of the present invention can be self-expanding or balloon-expanded, i.e. in different embodiments the stent of the present invention can be either self-expanding or balloon-expanded or a combination thereof wherein at least one stent element is balloon-expanded and at least one stent element is self-expandable. Moreover, the present invention can be modified to be used in various body lumens including highly tortuous and distal vasculature as well as to create whole or portions of other medical devices or markers placed on such devices.

Other features and advantages of the present invention will become more apparent from the following detailed description of the invention when taken in conjunction with the accompanying exemplary drawings.

### BRIEF DESCRIPTION OF THE DRAWINGS

FIGURE 1 illustrates a method of forming a ring.
FIG. 2 is a side view of a metallic core wire or tube covered by a polymeric tube.
FIGS. 3 and 4 illustrate a method of manufacturing a ring from a metallic core wire or tube covered with a polymeric tube.
FIG. 5 is a perspective illustrative view of the metallic core wire preformed to result in a cylindrical crown.
FIG. 6 is a perspective view of the metallic core wire preformed to result in a cylindrical crown and covered with a polymeric tube.
FIG. 7 is a perspective view of a cylindrical crown of the present invention
FIG. 8 is a perspective view of two cylindrical crowns aligned along a longitudinal axis.
FIG. 9 illustrates a partial view of a peak of a first cylindrical crown connected to a valley of a second cylindrical crown by a fist embodiment of a polymeric connector.
FIG. 10 illustrates a partial view of a peak of a first cylindrical crown connected to a valley of a second cylindrical crown by another embodiment of a polymeric connector.
FIG. 11 is an illustrative partial view of a crown comprising micro-pores.
FIGS. 12, 13 and 14 illustrate partial views of two crowns connected in different embodiments.
FIG. 15 illustrates a partial view of a stent formed from a first stent segment with a first web pattern and a second stent segment being a cylindrical crown according to the present invention.
FIG. 16 illustrates a view of a stent formed from 3 crowns aligned along a longitudinal axis of the stent and connected to each other vial polymeric connectors.

### DETAILED DESCRIPTION OF THE PREFERRED EMBODIMENTS

The present invention refers to an intravascular stent, consisting of at least two stent elements which are expandable from a first compressed delivery diameter to a second expanded implanted diameter wherein at least one stent element is a cylindrical crown, the cylindrical crown being aligned along a longitudinal axis of the stent and attached to the other at least one stent element by at least one connector, wherein the crown comprises a metallic core and a polymeric cover over the core, and wherein the at least one connector is formed from a polymeric material.

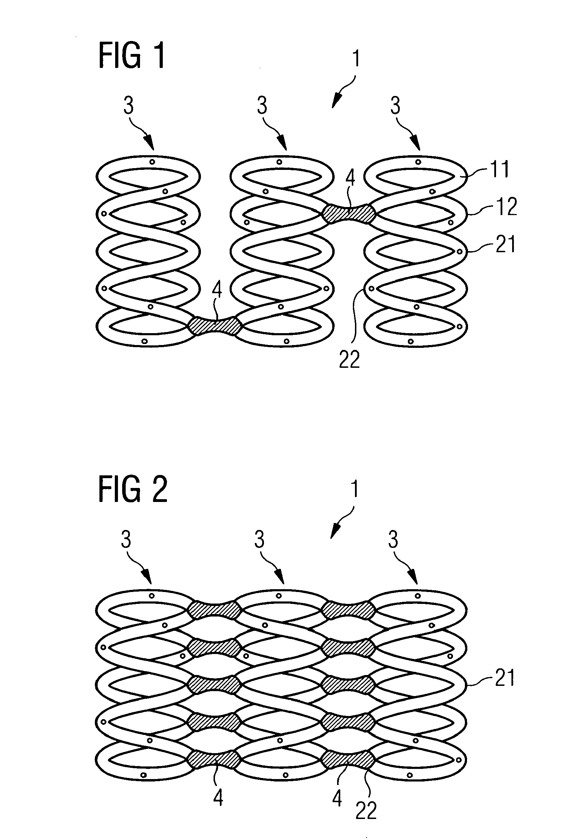

Figure 1 illustrates a stent (1) incorporating the features of the invention. The stent (1) generally comprises a plurality of radially expandable cylindrical crowns (3) which are aligned along a longitudinal axis of the stent and attached to each other by polymeric connectors (4). As depicted in Figure 1 the cylindrical crowns (3) can be formed with undulations having peaks (21) and valleys (22) generally formed as U or V members. Each radially expandable cylindrical crown (3) of the stent (1) may substantially independently expanded to some degree relative to adjacent crowns and thus the stent can have for example but not limitation a tapered, a barrel shaped or a dog bone shaped configuration upon delivery to suit the demands of the vessel configuration at the delivery site within the body lumen. Each crown (3) comprises a metallic core (11) and a polymeric cover (12) over the core (11).

Whereas the stent (1) illustrated in Fig. 1 has a so called open cell configuration, i.e. neighbouring crowns are connected to each other by a limited number of connectors at for example but not limitation every second, third, forth, fifth or sixth peak or valley of the crown, Fig. 2 illustrates an alternative stent (1) according to the present invention with a so called closed cell configuration, i.e. adjacent cylindrical crowns (3) are connected by connectors (4) at each repeating peak (21) or valley (22).

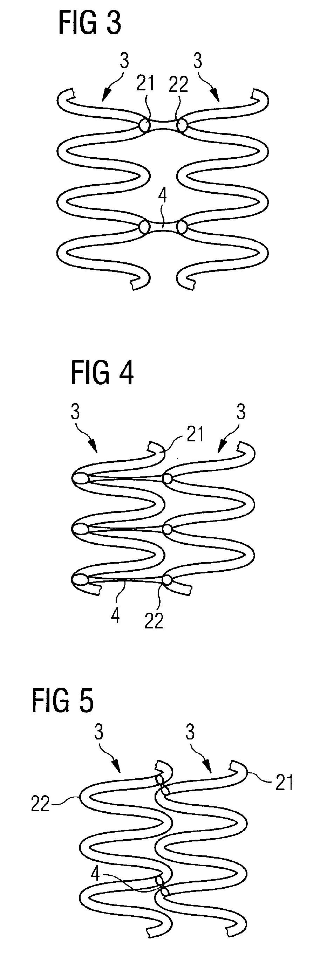

To provide the desired flexibility the peaks (21) of the first crown (3) and the valleys (22) of the neighbouring crown (3) can be axially aligned to each other as exemplarily depicted in Figure 3. In the embodiment shown in Figure 3 connectors (4) connect the peak (21) of a first crown (3') to the valley (22) of a second crown (3"). In an alternative embodiment as illustrated in Fig. 4 the peaks (21) and valleys (22) of two neighbouring crowns are axially aligned to each other and the peaks (21) or valley (22), respectively, of adjacent cylindrical crowns (3) are connected to each other by respectively longer connectors (4). In a further embodiment illustrated in Fig. 5 the peaks (21) of neighbouring cylindrical crowns (3) can be positioned laterally offset to each other to allow improved or excellent bending of the stent.

The properties of the stent (1) may further be varied by alteration of the pattern of the cylindrical crowns. Generally, the cylindrical crowns can be formed with undulations having peaks and valleys generally formed as U, V or W members. However, the cylindrical crowns may be of different shapes known in the art like of the shape of sine-waves, coils, z-shapes, or bowled shapes depending on the structural requirements.

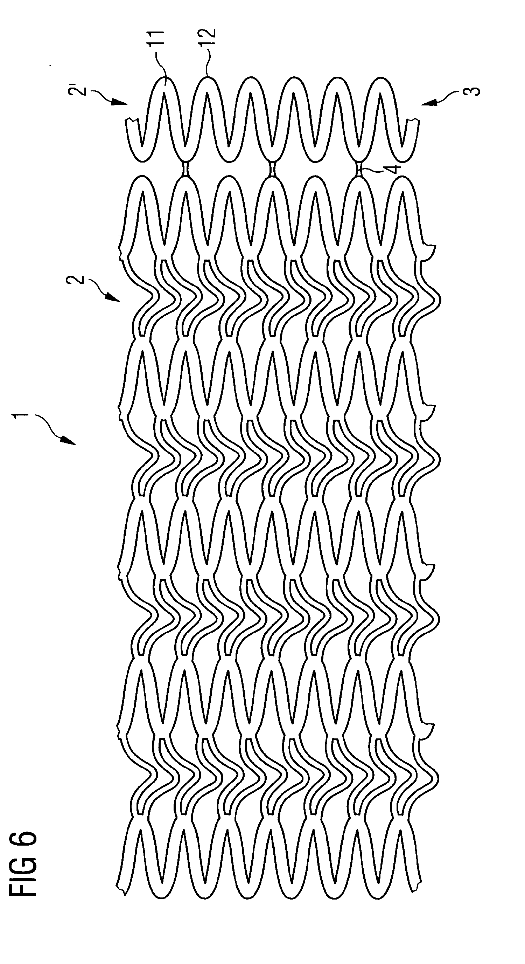

Figure 6 illustrates an alternative embodiment of the stent (1) of the present invention. The stent (1) comprises at least two stent elements (2 and 2') which are expandable from a first compressed delivery diameter to a second expanded implanted diameter wherein at least one stent element (2') is a cylindrical crown (3). The cylindrical crown (3) is aligned along a longitudinal axis of the stent and attached to the other at least one stent element (2) by at least one connector (3). The crown (3) comprises a metallic core (11) and a polymeric cover (12) over the core, and wherein the at least one connector (4) is formed from a polymeric material.

The stent element (2) which is different from the cylindrical crown (3) can have the configuration and stent pattern of any other stent known in the art. It may be self-expanding or balloon-expandable and may be formed from any known stent material. It may be covered with a membrane or graft material to result in a stent graft portion either permeable or impermeable to blood and/or may optionally be coated with a polymer and/or a pharmaceutically active substance.

Various configurations of combinations of a conventional stent segment (2') and the cylindrical crowns (3) of the present invention suit different needs in medical treatments of patients. Thus, it is obvious to the person skilled in the art that a conventional stent segment (2) may be connected to one or more cylindrical crown of the present invention on one or both stent ends. In an other embodiment conventional stent segments (2) and cylindrical crowns (3) are alternating.

In a further embodiment of the present invention the conventional stent segment (2) may include an opening for a side branch or may be bifurcated to suit the treatment of bifurcations. In the latter example, cylindrical crowns of the present invention may be positioned at all three stent ends or only at one or two.

Referring now back to Figure 1, the metallic core (11) of the cylindrical crown (3) may be formed for example but not limitation from a metallic wire, a band, a ring, or a tube. The metallic core determines the shape of the crown as well as the elastic properties and radial strength of the stent of the present invention. Thus, depending on the material used for the metallic core, the stent may be self-expanding or balloon-expandable, or a combination thereof wherein at least one stent element or crown is balloon-expandable and at least one stent element or crown is self-expandable.

Accordingly, the metallic core of the expandable crowns may be made from stainless steel, cobalt-chromium, tantalum, niobium, titanium, gold, platinum, iridium or alloys thereof or from a shape-memory material which is a superelastic material such as e.g. a nickel titanium alloy. In a preferred embodiment the stent is made from an MRI-compatible material like eg. CoCr alloys or NbTa alloys.

In a preferred embodiment at least one of the cylindrical crowns may include a material therein to enhance the radiopacity of the stent. In one example the metallic core may be made from a material with enhanced radiopacity or the metallic core may include portions of radiopaque material either incorporated into the metallic core or attached to the metallic core like for example by a radiopaque cover, by an insertion of a radiopaque material into the core or by addition of radipopaque markers to the core.

The polymeric cover over the metallic core of the cylindrical crown encloses the metal core completely. In a preferred embodiment the polymeric cover is made from a polymer tube which is imposed on the metallic core in a loose-fitted way. In another embodiment the polymeric cover may also be sintered onto the metallic core to result in a tight fit of polymeric cover and metallic core. Alternatively, the polymeric cover tube may be formed by rolling a flat polymeric sheet over the metallic core of the cylindrical crown and providing a longitudinal bond to form the crown. In further alternative embodiments the polymeric cover is formed by spinning, coiling, weaving or knitting a polymeric wire or thread over the metallic core.

The polymeric cover generally can be made from various polymers including polyurethanes, polyesters, polyamides, copolymers, block copolymers and blends or multi layers thereof. Further, the cover may be liquid tight and uniform or can be micro porous, wherein the pores can be material inherent like, e.g. in ePTFE membranes or spun, knitted or weaved membranes or the pores can be created by punching holes into the membranes by suitable means, like e.g. laser.

Suitable polymer material for forming the polymeric cover is taken from the group of polymers consisting of polyurethanes, polyolefins, polyesters, polyamides, flouropolymers and their co-polymers (e.g., PTFE ePTFE), polyetherurethanes, polyesterurethanes, silicone, thermoplastic elastomer (e.g., C-flex), polyether-amide thermoplastic elastomer (e.g., Pebax), fluoroelastomers, fluorosilicone elastomer, styrene-butadiene-styrene rubber, styrene-isoprene-styrene rubber, polyisoprene, neoprene (polychloroprene), polybutadienne-ethylene-propylene elastomer, chlorosulfonated polyethylene elastomer, butyl rubber, polysulfide elastomer, polyacrylate elastomer, nitrile rubber, a family of elastomers composed of 10 styrene, ethylene, propylene, aliphatic polycarbonate polyurethane, polymers augmented with antioxidents, polymers augmented with image enhancing materials, polymers having a proton (H+) core, polymers augmented with protons (H+), butadiene and isoprene (e.g., Kraton) and polyester thermoplastic elastomer (e.g., Hytrel).

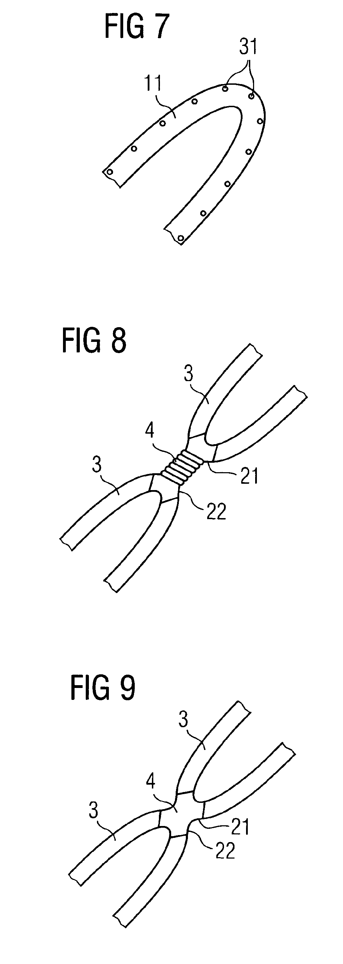

The polymeric connectors connecting the cylindrical crown to another stent segment can be made from a polymeric band, tube or wire or threat, cutted from a sheath or a tube or may be knitted, woven, braided or bound. The polymeric connectors may have any shape of known connector designs. The connector may be for example and not limitation straight, in form of a spiral, a S-curve, a sinusoidal curve, or may be hinged. The connector is fixed to the cylindrical crown an the stent segment by suitable means including knotting, gluing or welding. Figure 9 depicts one embodiment of a polymeric connector comprising a polymeric band welded around the peak (21) and valley (22) of two neighbouring cylindrical crowns (3). In an alternative embodiment illustrated in Figure 8, the connector is made from a polymeric thread which is twisted around the peak (21) and valley (22) of two neighbouring cylindrical crowns (3). By varying the length of the connectors, the longitudinal flexibility of the stent of the present invention can be easily controlled. Longer connectors can for example be employed to couple the outer cylindrivcal crowns to the stent to provide a maximum of flexibility at the stent ends, while shorter connectors are employed in the central stent area to ensure sufficient wall coverage of the stent in its middle portion.

Suitable polymers to form the connectors are taken from the group of polymers listed above as suitable to form the polymeric cover of the cylindrical crowns. The polymer the connectors are made from may be different from the polymer used for the polymeric cover or may be the same polymer. In a preferred embodiment of the present invention the polymeric cover is made from an ePTFE membrane and the connectors from PTFE as PTFE is highly biocompatible, electrically neutral due to its dielectric properties and is an excellent drug carrier. In a even more preferred embodiment, the stent consists of cylindrical crowns that are completely covered with sintered ePTFE as the polymer cover and the crowns are connected by knots of PTFE threads.

The polymeric connectors provide longitudinal and flexural flexibility of the stent superior to the flexibility achievable with metal connectors in conventional stents, thus facilitating delivery through tortuous body lumens. The crowns on the other hand maintain sufficient radial strength to maintain the patency of a vessel and to resist collapse. Accordingly, the stent of the present invention shows increased longitudinal flexibility combined with high radial strength.

In a further embodiment of the present invention the polymer of at least a portion of the polymeric cover or at least a portion of at least one marker may be dotted with radiopaque material like e.g. barium sulphate to enhance visibility of the stent under x-ray.

In an alternative embodiment of the present invention the connectors are formed from biodegradable, biocompatible polymers, like for example but not limitation Poly-L-lactic acid (PLLA), polyglycolic acid (PGA), poly (D, L-lactide/glycolide) copolymer (PDLA), and polycaprolactone (PCL). In a stent according to this embodiment the connectors will biodegrade upon delivery in the bodily lumen and leave behind single stent elements or cylindrical crowns thus leading to an increased longitudinal flexibility of the implanted stent upon deployment, avoiding any risk of connector failure over time.

In a preferred embodiment at least one cylindrical crown of the stent of the present invention is loaded or coated with a pharmaceutically active substance that may be eluted into the vessel subsequent to deployment of the stent in a controlled manner. Pharmaceutically active substances are generally employed to perform a variety of functions, from preventing blood clots or restenosis to promoting healing. As an example, a pharmaceutically active substance loaded into or coated on the cylindrical crown can inhibit the activity of vascular smooth muscle cells. More specifically, the pharmaceutically active substance is aimed at inhibiting abnormal or inappropriate migration and proliferation of smooth muscle cells. The pharmaceutically active substance can also include any substance capable of exerting a therapeutic or prophylactic effect in the practice of the present invention. The pharmaceutically active substance can also be for enhancing wound healing in a vascular site or improving the structural and elastic properties of the vascular site. The dosage or concentration of the pharmaceutically active substance required to produce a favourable therapeutic effect should be less than the level at which the pharmaceutically active substance produces toxic effects and greater than the level at which non-therapeutic results are obtained. The dosage or concentration of the active agent required to inhibit the desired cellular activity of the vascular region can depend upon factors such as the particular circumstances of the patient; the nature of the trauma; the nature of the therapy desired; the time over which the ingredient administered resides at the vascular site; and if other therapeutic agents are employed, the nature and type of the substance or combination of substances. Therapeutic effective dosages can be determined empirically, for example by infusing vessels from suitable animal model systems and using immuno histochemical, fluorescent or electron microscopy methods to detect the agent and its effects, or by conducting suitable in vitro studies. Standard pharmacological test procedures to determine dosages are understood by one of ordinary skill in the art.

Examples of pharmaceutically active substance include rapamycin, actinomycin D (ActD), or derivatives and analogs thereof or Cosmegen. Synonyms of actinopmycin D include dactinomycin, actinomycin IV, actinomycin 11, actinomycin X1, and actinomycin C1. Examples of pharmaceutically active substance include other antiproliferative substances as well as antineoplastic, antinflammatory, antiplatelet, anticoagulant, antifibrin, antithomobin, antimitotic, antibiotic, and antioxidant substances. Examples of antineoplastics include taxol (paclitaxel and docetaxel). Examples of antiplatelets" anticoagulants, antifibrins, and antithrombins include sodium heparin, low molecular weight heparin, hirudin, argatroban, forskolin, vapiprost, prostacyclin andprostacyclin analogs, dextran, D-phe-pro-arg-chloromethylketone (synthetic antithrombin), dipyridamole, glycoprotein, IIIb/IIIa platelet membrane receptor antagonist, recombinant hirudin, thrombin inhibitor, and 7E-3B. Examples of antimitotic agents include methotrexate, azathioprine, vincristine, vinblastine, fluorouracil, adriamycin, and mutamycin. Examples of cytostatic or antiproliferative agents include angiopeptin (a somatostatin analog), angiotensin converting enzyme inhibitors such as Captopril, Cilazapril, or Lisinopril; calcium channel blockers (such as Nifedipine), colchicine fibroblast growth factor (FGF) antagonists, fish oil (omega 3-fatty acid), histamine antagonist, Lovastatin (an inhibitor of HMG-CoA reductase, a cholesterol lowering drug), monoclonal antibodies (such as PDGF receptors), nitroprusside, phosphodiesterase inhibitors, prostaglandin inhibitor, Seramin (a PDGF antagonist), serotonin blockers, steroids, thioprotease inhibitors, triazolopynmidine (a PDGF antagonist), and nitric oxide.

Other therapeutic substances or agents which may be appropriate include alpha-interferon, genetically engineered epithelial cells, dexamethasone or means for gene therapy.

The pharmaceutically active substance may be added to the cylindrical crown in many different ways. For example and not limitation, the polymeric cover of the cylindrical crown can be coated with the pharmaceutically active substance according to any coating technique known in the art, or the polymeric cover material can be loaded with the pharmaceutically active substance before or after the process of covering the metallic core.

In one embodiment of the present invention as depicted in Figure 7 the metallic core (11) is made from a hollow tube having micropores (31) in the tube wall that communicate with the lumen. The tube may then be filled with a liquid, a foam, a gel, a powder, or a polymer containing the pharmaceutically active substance. The pharmaceutically active substance may elute through the micropores of the metallic core into the polymeric cover. Depending on the characteristics and porosity of the polymeric cover the pharmaceutically active substance will the subsequently be eluted into the vessel wall upon deployment of the stent in the vessel. It is obvious to the person skilled in the art that the size, shape and distribution of the micropores may vary depending of the desired drug-elution profile.

In a further alternative embodiment, where the polymeric cover is arranged in a loose fit over the metallic core of the cylindrical crown, the pharmaceutically active substance is loaded into the interspace between the metallic core and the polymeric cover and will the be eluted through the polymeric cover upon deployment of the stent.

In a further embodiment of the present invention a closed or perforated polymer layer (e.g. made from ePTFE) may be attached to the covered cylindrical crowns to provide a single layer graft. The resulting stent graft displays excellent binding capabilities between stent and graft material (polymer layer).

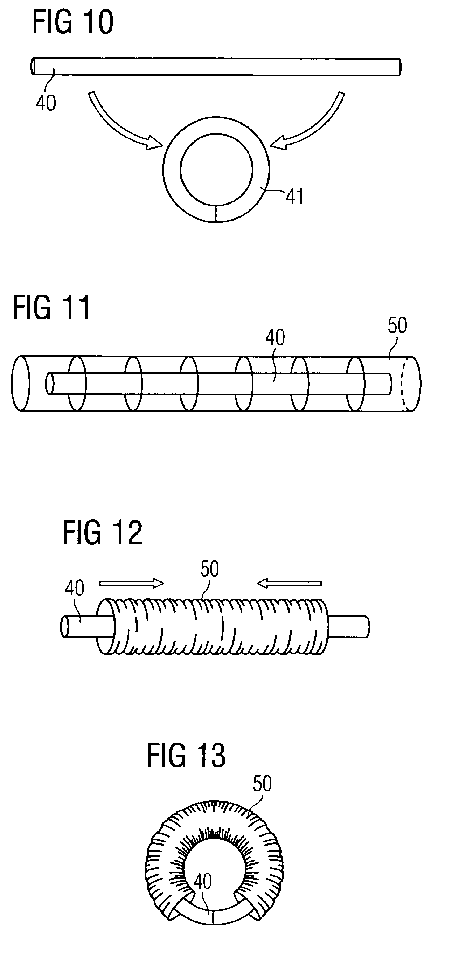

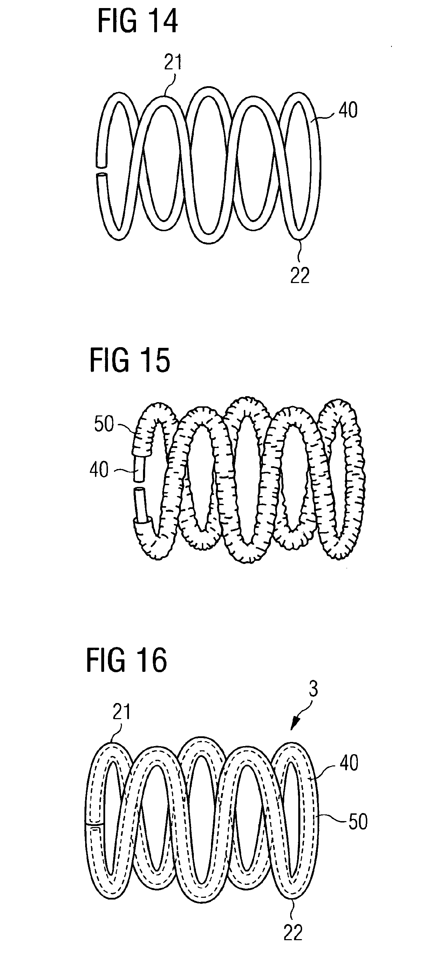

One method of making a stent according to the present invention is illustrated in Figures 10 to 16. A metallic core (40) like a wire, a tube, or a band as well as a polymeric sheath (50), preferably a polymeric tube, are provided. In a first step as depicted in Figure 11, the polymeric sheath or tube (50) which has a slightly bigger inner circumference than the outer circumference of the metallic core is imposed on the metallic core (40). To form a cylindrical crown the polymeric cover tube (50) is slightly compressed in longitudinal direction to free the ends of the metallic core (see Figure 12). Subsequently, the ends of the metallic core are connected to each other by suitable means like gluing or welding and a closed metal ring is formed as depicted in Figure 13. In a next step the ends of the polymeric tube are also circumferentially closed by suitable means including welding or gluing to result in a closed ring structure that is completely covered with the polymeric cover. The covered ring is then formed to have a crown-like structure with peaks (21) and valleys (22) as depicted in Figure 16.

In order to avoid damage to the polymeric tube, in an alternative embodiment illustrated in Figure 14 the metallic core wire or tube (40) may be formed to a crown shape with peaks (21) and valleys (22) und the covering by the polymeric cover or tube (50) may then be performed after the crown-shape is imparted on the metallic core (40) as shown in Figure 15. According to the previous embodiment the final cylindrical crown is the formed of the covered metallic core by first connecting the free ends of the metallic core to form a closed configuration and secondly by connecting the free ends of the polymeric cover to form a ring-closed structure around the core.

In further embodiments according to the present invention the polymeric cover tube may also be formed by rolling a flat polymeric sheet over the metallic core of the cylindrical crown and providing a longitudinal bond to form the crown. Other methods of forming the polymeric cover include spinning, coiling, weaving or knitting a polymeric wire around the metallic core to form the covered cylindrical crown.

The polymeric cover may be placed in a loose-fit configuration over the metallic core, may be tightly fitted to the metallic core or may be shrinked onto the metallic core to result in a tight fit between metallic core and polymeric cover, by e.g. sintering the polymeric cover. If for example an ePTFE tube is employed as polymeric cover, the ePTFE may be sintered onto the metallic core at a temperature of approximately 340°. During this sintering process the ePTFE tube shrinks onto the metallic core resuting in a tight fit between ePTFE and metallic core.

Once the cylindrical crown is manufactured the crown is aligned to at least one other stent element along a longitudinal axis of the stent and the crown is attached to the other at least one stent element by at least one polymeric connector.

In one embodiment, the crowns are connected to each other at their peaks. In one embodiment the peaks of two crowns are connected via an ePTFE foil which may be knotted or glued or twisted. By adjusting length, consistence or thickness, or density of the ePTFE connectors, the flexibility and wall coverage of the resulting stents may be varied according to the clinical needs. In another embodiment the crowns are connected in a way that the peaks do not face each other but have an offset in longitudinal direction to avoid any contact of the neighboring crowns even in case the stent undergoes a narrow bending.

The stent of the present invention provides a highly flexible but radially strong, extremely biocompatible and electrically neutral structure which can be easily loaded with a therapeutic agent prior to implantation but which also can also be reloaded with a pharmaceutically active agent post implantation.

While the invention has been described in connection with certain disclosed embodiments, it is not intended to limit the scope of the invention to the particular forms set forth, but, on the contrary it is intended to cover all such alternatives, modifications, and equivalents as may be included in the spirit and scope of the invention as defined by the appended claims.

## Claims

1. An intravascular stent, comprising at least two stent elements which are expandable from a first compressed delivery diameter to a second expanded implanted diameter wherein at least one stent element is a cylindrical crown, the cylindrical crown being aligned along a longitudinal axis of the stent and attached to the other at least one stent element by at least one connector, wherein the crown comprises a metallic core and a polymeric cover over the core, and wherein the at least one connector is formed from a polymeric material.

2. An intravascular stent according to claim 1, comprising a plurality of cylindrical crowns being expandable from a first compressed delivery diameter to a second expanded implanted diameter, the cylindrical crowns being aligned along a longitudinal axis of the stent and attached to each other by at least one connector, wherein the crowns comprise a metallic core and a polymeric cover over the core, and wherein the at least one connectors is formed from a polymeric material.

3. The stent of claim 1 or 2, wherein the metallic core is formed from either a wire, a hollow tube, a band, or a ring.

4. The stent according to any of claims 1 to 3, wherein the metallic material forming the core of the cylindrical crown is taken from the group of alloys comprising stainless steel, titanium, tantalum, nickel titanium, cobalt-chromium, gold, paladium, platinum and iridium.

5. The stent according to any of claims 1 to 4, wherein the cylindrical crowns include a material therein to enhance the radiopacity of the stent.

6. The stent according to any of claims 1 to 5, wherein the stent may be balloon expandable or self-expanding or a combination thereof.

7. The stent according to any of claims 1 to 6 wherein the polymer material forming the polymeric cover is taken from the group of polymers consisting of polyurethanes, polyolefins, polyesters, polyamides, flouropolymers and their copolymers (e.g., PTFE ePTFE), polyetherurethanes, polyesterurethanes, silicone, thermoplastic elastomer (e.g., C-flex), polyether-amide thermoplastic elastomer (e.g., Pebax), fluoroelastomers, fluorosilicone elastomer, styrene-butadiene-styrene rubber, styrene-isoprene-styrene rubber, polyisoprene, neoprene (polychloroprene), polybutadienne-ethylene-propylene elastomer, chlorosulfonated polyethylene elastomer, butyl rubber, polysulfide elastomer, polyacrylate elastomer, nitrile rubber, a family of elastomers composed of 10 styrene, ethylene, propylene, aliphatic polycarbonate polyurethane, polymers augmented with antioxidents, polymers augmented with image enhancing materials, polymers having a proton (H+) core, polymers augmented with protons (H+), butadiene and isoprene (e.g., Kraton) and polyester thermoplastic elastomer (e.g., Hytrel).

8. The stent according to any of claims 1 to 7, wherein the polymeric cover is sintered or shrinked onto the metallic core to result in a tight fit between metallic core and cover.

9. The stent according to any of claims 1 to 8, wherein the polymeric cover has a porous structure.

10. The stent according to one of the previous claims 1 to 9, wherein the polymeric connectors are formed from a biodegradable polymer.

11. The stent according to any of claims 1 to 10 comprising a therapeutic agent releasably loaded to the stent.

12. The stent of claim 11, wherein the polymeric cover is loaded with a pharmaceutically active substance or coated with a pharmaceutically active substance.

13. The stent of claim 11, wherein the the hollow tube is filled with a pharmaceutically active substance and comprises micropores allowing controlled release of the pharmaceutically active substance into the blood or vessel surface.

14. A method for forming an intravascular stent, comprising the steps of:
providing a metallic core;
providing a polymeric tube;
imposing the polymeric tube on the metallic core to give a polymeric cover;
forming a circular crown by first connecting the free ends of the metallic core to form a ring-shape;
and second by connecting the free ends of the polymeric cover to form a ring-closed structure around the core;
aligning the crown to at least on other stent element along a longitudinal axis of the stent;
and attaching the crown to the other at least one stent element by at least one connector,
the connector consisting of a polymeric material.
